# EUROPEAN PATENT SPECIFICATION

(11) **EP 2 695 621 B1**
(45) Date of publication and mention of the grant of the patent: **12.08.2020**
(21) Application number: 12768019.7
(22) Date of filing: 23.03.2012
(51) Int. Cl.: A61K 47/36, A61K 9/70, A61K 31/5575, A61K 45/00, A61K 47/34, A61P 27/02, A61P 27/06

(54) **PHARMACEUTICAL PREPARATION**
PHARMAZEUTISCHE ZUBEREITUNG
PRÉPARATION PHARMACEUTIQUE

(30) Priority: 08.04.2011 JP 2011086402
(43) Date of publication of application: 12.02.2014
(73) Proprietor: Nanotheta Co, Ltd., Tokyo 169-0051 (JP); Toray Industries, Inc., Tokyo, 103-8666 (JP)
(72) Inventor: TAKEOKA Shinji, Tokyo 1580094 (JP); KASHIWAGI Kenji, Kofu-shi Yamanashi 400-8510 (JP); FUJIE Toshinori, Nishitokyo-shi Tokyo 188-0014 (JP); SAITO Akihiro, Koga-shi Ibaraki 306-0023 (JP); HANIUDA Hiroki, Suzaka-shi Nagano 382-0041 (JP)
(74) Representative: Mewburn Ellis LLP
(86) International application number: PCT/JP2012/057519
(87) International publication number: WO 2012/137610

(56) References cited:
- EP-A1- 2 082 869
- WO-A1-2005/115496
- WO-A1-2008/050913
- WO-A2-2009/117473
- WO-A2-2009/137085
- WO-A2-2010/021973
- JP-A- 2007 523 911
- JP-A- 2009 521 433
- JP-A- 2010 525 795
- SHINJI TAKEOKA ET AL: "Development of biodegradable nanosheets as nanoadhesive plaster", PURE AND APPLIED CHEMISTRY, vol. 80, no. 11, 1 January 2008 (2008-01-01), XP55130979, ISSN: 0033-4545, DOI: 10.1351/pac200880112259
- MACDONALD M ET AL: "Release of a model protein from biodegradable self assembled films for surface delivery applications", JOURNAL OF CONTROLLED RELEASE, ELSEVIER, AMSTERDAM, NL, vol. 131, no. 3, 12 November 2008 (2008-11-12), pages 228-234, XP025583901, ISSN: 0168-3659, DOI: 10.1016/J.JCONREL.2008.07.032 [retrieved on 2008-07-30]
- YE S ET AL: "Deposition temperature effect on release rate of indomethacin microcrystals from microcapsules of layer-by-layer assembled chitosan and alginate multilayer films", JOURNAL OF CONTROLLED RELEASE, ELSEVIER, AMSTERDAM, NL, vol. 106, no. 3, 2 September 2005 (2005-09-02), pages 319-328, XP027664173, ISSN: 0168-3659 [retrieved on 2005-09-02]
- Anonymous: "Martindale: The Complete Drug Reference", 1999, Pharmaceutical Press, XP002727907, * page 1419 *
- AKIHIRO SAITO ET AL.: 'Kobunshi Nanosheet no Bussei to Iryo Tenkai' EXPECTED MATERIALS FOR THE FUTURE vol. 11, no. 3, 10 March 2011, pages 24 - 28, XP008170632
- SHINJI TAKEOKA: 'Preparation, properties, and applications of polymer ultra-thin films (nanosheets)' OYO BUTSURI vol. 80, no. 2, February 2011, pages 133 - 136, XP008170630
- TOSHINOBU FUJIEDA ET AL.: 'Development of polymeric nanosheets for the biomedical application' HYOMEN vol. 48, no. 7, 01 July 2010, pages 211 - 219, XP008170631

## Description

### TECHNICAL FIELD

The present invention relates to a pharmaceutical preparation that has a longer duration of drug action with a single dose.

### BACKGROUND ART

Glaucoma is a disease that is one of the leading causes of blindness in ophthalmology, which affects optic nerve mainly due to elevation of intraocular pressure and leads to abnormal visual field or decrease in vision. Glaucoma is principally treated by lowering the intraocular pressure. Exemplary methods for lowering the intraocular pressure include drug therapy, laser therapy, treatment by surgical operation and the like.

For drug therapy, drugs such as a beta-blocker, a prostaglandin-related drug, a carbonic anhydrase inhibitor, a cholinergic agonist and an epinephrine-related drug are used. These drugs are administrated as eyedrops to an eye of a patient.

Eyedrops, however, have a problem of a short duration of action with a single dose. Accordingly, a pharmaceutical preparation that has a longer duration of drug action with a single dose has been demanded.

Meanwhile, a thin-film polymeric structure with an arbitrary shape and a method for preparing the same are described in Patent Documents 1 and 2.

In addition, Patent Document 3 describes a kit of sustained-drug release contact lenses. Since contact lenses in this kit are made of a hydrogel material that has a substantial thickness that causes uncomfortable feeling upon wearing them, they may be unwearable for some patients.

Non-Patent Document 1 describes the effect of applying an antibiotic-loaded nanosheet for perforative peritonitis.

Non-Patent Document 2 describes contact lenses that sustainably releases an allergy medicine.

### PRIOR ART DOCUMENTS

### [Patent Documents]

[Patent Document 1] WO2006/025592 (pamphlet)
[Patent Document 2] WO2008/050913 (pamphlet)
[Patent Document 3] Patent No. 4268912 (specification)

### [Non-Patent Documents]

[Non-Patent Document 1] Biomaterials 31(2010), 6269-6278
[Non-Patent Document 2] Website of SEED Co., Ltd., news release, "1. Study on contact lenses that sustainably release allergy medicine", attachment of 'Report on the achievement of joint research with Senju Pharmaceutical Co., Ltd.: Drug Delivery System (DDS) in ophthalmic field', [on line], April 28, 2010 [searched on March 22, 2011], Internet <URL: http://eir.eol.cojp/EIRNavi/DocumentNavigator/ENavigatorBody.aspx?cat=tdnet&sid=791 913&code=7743&ln=ja&disp=simple>.

### SUMMARY OF THE INVENTION

### Problem to be solved by the Invention

The present invention has an objective of providing a pharmaceutical preparation or the like comprising a thin film loaded with a drug to be instilled into an eye.

### Means for Solving the Problem

In order to solve the above-described problem, the present inventors have gone through intensive studies and figured out that use of a pharmaceutical preparation comprising a layer-by-layer thin film loaded with a drug that is to be instilled into an eye can prolong the duration of drug action with a single dose, thereby accomplishing the present invention.

Specifically, the present invention provides the following pharmaceutical preparation and else.
[1] A pharmaceutical preparation for use in a method of treating glaucoma by application to the eye, said preparation comprising a layer-by-layer thin film that is producible by alternately layering a polycation and a polyanion, and one or more type(s) of drug loaded onto the layer-by-layer thin film or between the layers, which is/are to be instilled into the eye, the ocular surface, or the periocular area; wherein
   the one or more type(s) of drug is/are for treating glaucoma; and
   wherein the polycation is selected from the group consisting of chitosan, polylysine, polyarginine, polyhistidine, ionene, poly(quarterized pyridine), polymers of diallyldiakylammonium salt, and combinations thereof; and
   wherein the polyanion is selected from the group consisting of alginic acid, hyaluronic acid, chondroitin sulfate, polyglutamic acid, polymethacrylic acid, polyacrylic acid, polystyrene sulfonate and alkali salts thereof.
[2] The pharmaceutical preparation according to [1] above, wherein the polycation is chitosan.
[3] The pharmaceutical preparation according to either one of [1] and [2] above, wherein the polyanion is sodium alginate.
[4] The pharmaceutical preparation according to any one of [1] to [3] above, wherein the layer-by-layer thin film has a film thickness of 5 nm to 500 nm without the drug.
[5] The pharmaceutical preparation according to any one of [1] to [4] above, wherein the number of polycation and polyanion layers of the layer-by-layer thin film is 2 to 50.
[6] The pharmaceutical preparation according to any one of [1] to [5] above, wherein the shape of the layer-by-layer thin film is a quadrangle, a circle, an oval, a doughnut shape or a ring shape.
[7] The pharmaceutical preparation according to any one of [1] to [6] above, wherein the drug is loaded by being layered onto one surface of the layer-by-layer thin film.
[8] The pharmaceutical preparation according to any one of [1] to [7] above, wherein the drug is loaded at an amount of 0.25 µg/cm² to 25 µg/cm².
[9] The pharmaceutical preparation according to [1] above, wherein the one or more type(s) of drug for treating glaucoma is/are
   at least one type of drug selected from a group consisting of prostaglandins, beta blockers and carbonic anhydrase inhibitors.
[10] The pharmaceutical preparation according to [9] above, wherein the one or more types of drugs for treating glaucoma are Latanoprost.
[11] The pharmaceutical preparation according to any one of [1] to [10] above, wherein the layer-by-layer thin film further comprises a polyvinyl alcohol layer as a supporting film layer on at least one surface thereof.
[12] The pharmaceutical preparation according to [11] above, comprising the supporting film layer on the surface opposite to the surface layered with the drug.
[13] The pharmaceutical preparation according to [12] above, wherein the pharmaceutical preparation is applied to the eye by bringing the drug-layered surface into contact with the eye and then removing the supporting film on the opposite surface.
[14] The pharmaceutical preparation according to any one of [7] to [13] above, further comprising, on the drug-layered surface, a drug release control film formed from polyvinyl acetate, polylactic acid, polyglycolic acid, polycaprolactone or a copolymer of any combination thereof.
[15] The pharmaceutical preparation according to any one of [1] to [13] above, which is for use in a method in which a drug control film formed from polyvinyl acetate, polylactic acid, polyglycolic acid, polycaprolactone or a copolymer of any combination thereof to the eye, the layer-by-layer thin film loaded with the drug is applied on said drug control film.

### EFFECT OF THE INVENTION

The present invention provides a pharmaceutical preparation comprising a drug-loaded layer-by-layer thin film. A preferable embodiment of a pharmaceutical preparation of the present invention has prolonged duration of drug action with a single dose. Since a further preferable embodiment of a pharmaceutical preparation of the present invention uses a highly biocompatible material and the thickness of the layer-by-layer thin film is very thin and flexible, it has at least one effect selected from the followings: there is less concern upon wearing it, such as mattering perceived by the wearer or adverse effects such as bloodshot; there is no need of removal because of dissolution in safety; and the like. In addition, in another preferable embodiment of a pharmaceutical preparation of the present invention, the drug to be contained is not fixed to the layer-by-layer thin film via a chemical bond or the like, a broad range of drugs can be loaded onto the layer-by-layer thin film, and thus multiple agents can simultaneously be administered.

### BRIEF DESCRIPTION OF DRAWINGS

[Figure 1] A schematic view showing a layer-by-layer (LBL) thin film of the present invention.
[Figure 2] A schematic view showing a method for preparing a Latanoprost-loaded LBL nanosheet.
[Figure 3] Images of layer-by-layer (LBL) nanosheets observed with an atom force microscope (AFM). (a) shows an edge of the LbL nanosheet on a silicon substrate, where the silicon substrate surface is on the left hand side while the LbL nanosheet surface is on the right hand side. (b) shows the surface of the LbL nanosheet.
[Figure 4] Images of Latanoprost-loaded LBL nanosheets with AFM. (a) shows an edge of the Latanoprost-loaded LBL nanosheet on a silicon substrate, where the silicon substrate surface is on the left hand side while the Latanoprost-loaded nanosheet surface is on the right hand side (white areas represents Latanoprost). (b) shows the Latanoprost-loaded nanosheet surface (white areas represents Latanoprost).
[Figure 5] Schematic views showing an experimental maneuver for measuring the release behavior of a Latanoprost-loaded LBL nanosheet. (a) A drug is loaded on the upper surface of a LBL nanosheet. (b) A drug is loaded on the bottom surface of a LBL nanosheet.
[Figure 6] Schematic views showing the results from measuring the release behavior of the Latanoprost-loaded LBL nanosheet. (a) Latanoprost is loaded on the upper surface of the LBL nanosheet. (b) Latanoprost is loaded on the bottom surface of the LBL nanosheet.
[Figure 7] A graph showing changes in the intraocular pressure of a rat upon use of a Latanoprost-loaded LBL nanosheet. (p<0.05 vs. control)
[Figure 8] A graph showing changes in the intraocular pressure of a rat upon use of Latanoprost-unloaded LBL nanosheet.
[Figure 9] Pictures of cornea specimens on Day 1 following application of the Latanoprost-loaded LBL nanosheets onto the corneas, (a) rabbit and (b) rat.
[Figure 10] A graph showing changes in the Latanoprost concentration in the aqueous humor of a white rabbit upon use of a Latanoprost-loaded LBL nanosheet. The vertical axis (latanoprost conc.) represents the Latanoprost concentration while the horizontal axis (time) represents time after the application of Latanoprost.

### EMBODIMENTS FOR CARRYING OUT THE INVENTION

Hereinafter, the present invention will be described in detail. The scope of the present invention is not limited to these descriptions, and may be carried out according to a procedure other than the following examples.

Herein, a thin film may sometimes referred to as a "nanosheet".

### 1. General outline of the present invention

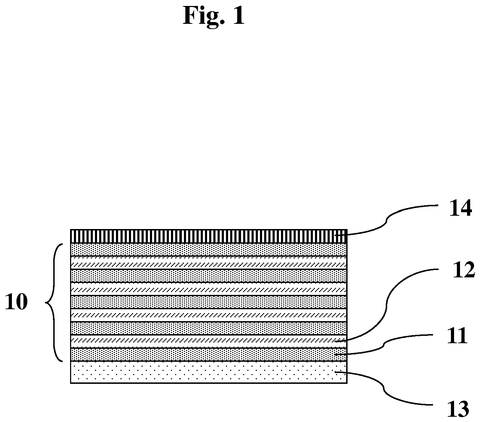

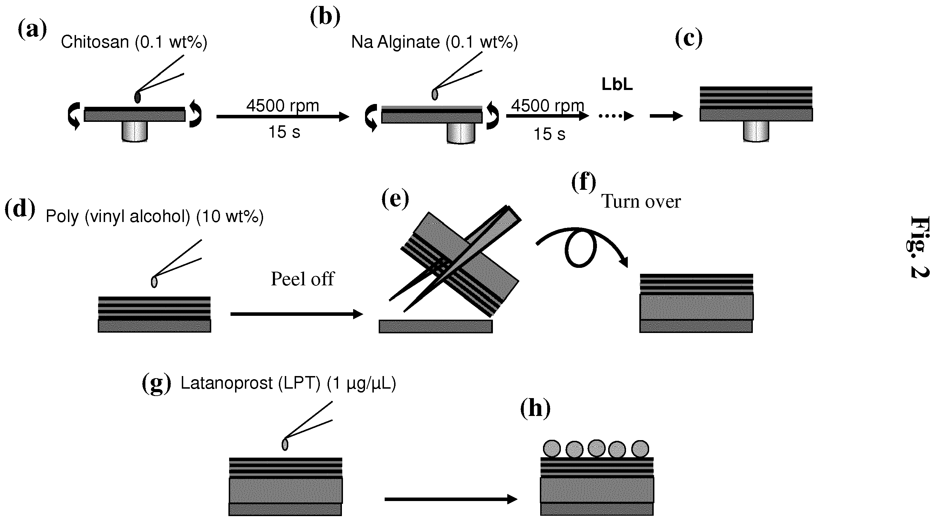

The present invention uses a pharmaceutical preparation comprising a thin film loaded with a drug that is to be instilled into an eye so as to prolong the duration of drug action with a single dose. The thin film is a layer-by-layer thin film (Figure 1), and may be formed, for example, by a spin coating process (Figure 2), a dipping process, a spray coating process or the like.

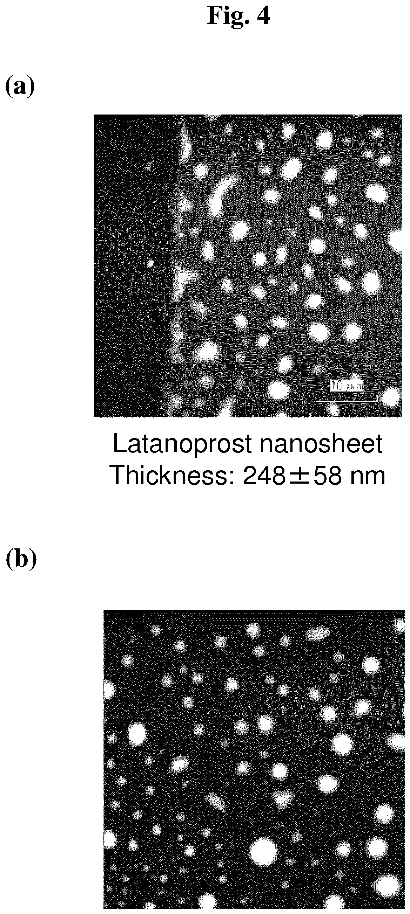

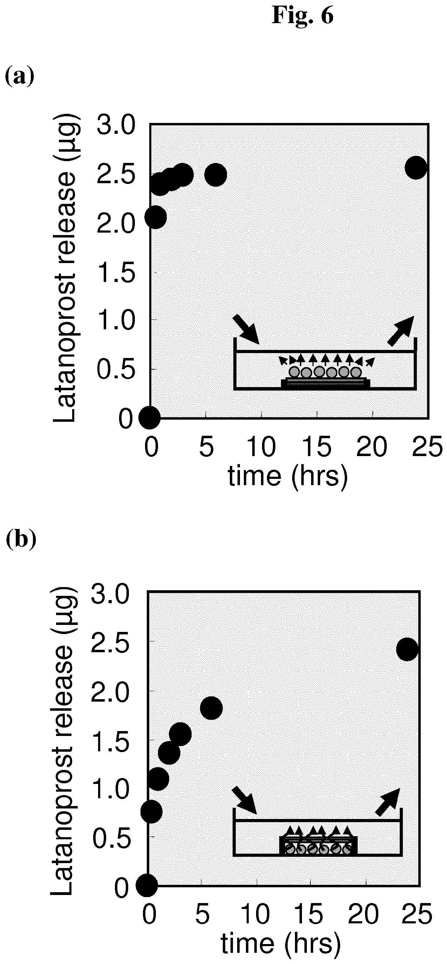

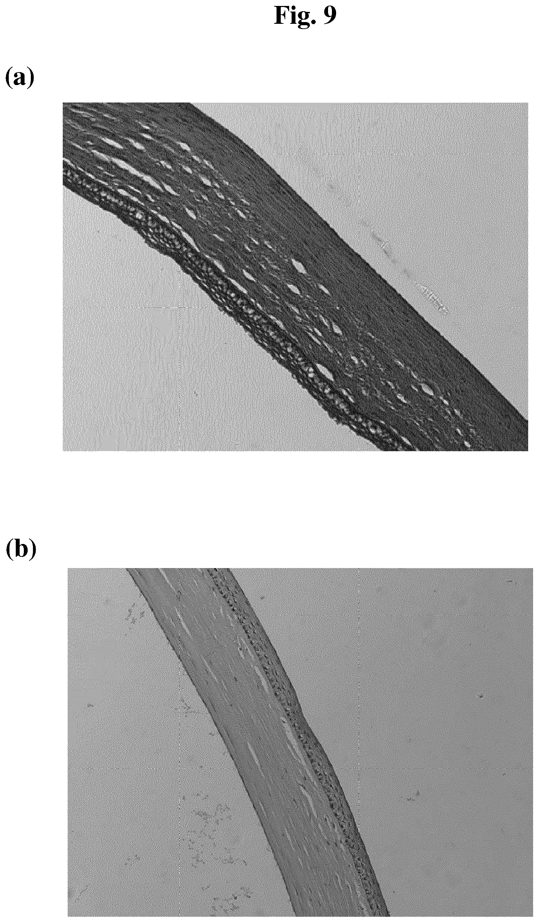

In a later-described example, a drug-loaded layer-by-layer thin film (Figures 1 and 4) was applied to the eyes and was able to prolong the duration of drug action with a single dose as compared to the use of eyedrops (Figures 6-8 and 10). Furthermore, in a later-described example, the drug-loaded layer-by-layer thin film was confirmed to have lower tissue toxicity (Figure 9).

### 2. Drug-loaded layer-by-layer thin film

As shown in Figure 1, a layer-by-layer (LBL) thin film 10 of the present invention is a thin film obtained by alternately layering polyelectrolyte layers with opposite charges (polycations 11 and polyanions 12). Additionally, a supporting film layer 13 may further be provided on the surface of the layer-by-layer thin film 10 of the present invention.

Furthermore, the layer-by-layer thin film 10 of the present invention is loaded with one or more types of drugs 14.

The polyelectrolytes used with the layer-by-layer thin film of the present invention are preferably biocompatible polyelectrolytes, and more preferably biocompatible and biodegradable polyelectrolytes.

Examples of polycations include chitosan, polylysine, polyarginine, polyhistidine, ionene, poly(quaternized pyridine) and polymers of diallyldialkylammonium salt. Polycation is preferably chitosan, polylysine, polyarginine or the like, and more preferably chitosan.

Examples of polyanions include alginic acid, hyaluronic acid, chondroitin sulfate, polyglutamic acid, polymethacrylic acid, polyacrylic acid, polystyrene sulfonate and alkali metal salts thereof (e.g., sodium alginate, sodium hyaluronate, sodium chondroitin sulfate, sodium polyglutamate, etc.). Polyanion is preferably sodium alginate, sodium hyaluronate, sodium chondroitin sulfate or the like, and more preferably sodium alginate.

A layer-by-layer thin film of the present invention may further comprise support film 13 such as a water-soluble film of polyvinyl alcohol, starch, polyelectrolytes or the like or a mesh-like film of polyethylene terephthalate, nylon, teflon, silk or the like on at least one of the surfaces (preferably one surface, and more preferably the surface opposite to the drug-layered surface). The polyelectrolytes may be polyelectrolytes used in a layer-by-layer technique. By providing a supporting film, the layer-by-layer thin film of the present invention can easily be handled. The water-soluble film may be dissolved, for example, with physiological saline after applying the layer-by-layer thin film onto the eye. The mesh-like film may be peeled off with tweezers or the like after application of the layer-by-layer thin film onto the eye.

The layer-by-layer thin film of the present invention may further comprise a layer of a polymer (e.g., polyethylene, polyolefin such as polypropylene, polyethylene terephthalate, cellophane), paper, fabric or the like as a protective film on at least one of the surfaces (preferably on both surfaces). By providing a protective film, the layer-by-layer thin film of the present invention can physically be protected from friction, impact, bend and the like.

The order of layering and the number of layers of the layer-by-layer thin film of the present invention are not particularly limited, and any order and any number of layers may be selected. The number of polycation and polyanion layers (the number of pairs of polycations and polyanions) is, for example, 2 to 50 layers (1 to 25 pairs), preferably 4 to 40 layers (2 to 20 pairs), and more preferably 6 to 30 layers (3 to 15 pairs) in total.

The shape of the layer-by-layer thin film of the present invention may be arbitrary, including, for example, a quadrangle, a circle, an oval, a ribbon shape, a string shape, a doughnut shape, a ring shape and the like, and preferably a quadrangle, a circle, an oval, a doughnut shape, a ring shape or the like.

Furthermore, the layer-by-layer thin film of the present invention may have a curved surface that fits the curvature of an eyeball. In some of the embodiments of the present invention, the supporting film may have a curved surface that fits the curvature of an eyeball. In this case, the protective film may be a sac.

As shown in Figure 1, the layer-by-layer thin film 10 of the present invention is loaded with an arbitrary drug 14. Although the drug 14 is layered on a surface of the layer-by-layer thin film 10 of the present invention in Figure 1, the present invention is not particularly limited to this case and a drug may be layered between layers. In the case where a drug is layered between layers of a layer-by-layer thin film, the duration of drug action is expected to prolong.

Even when a drug is layered on the surface or between the layers of the layer-by-layer thin film upon production, sometimes the drug may subsequently penetrate through the entire layer-by-layer film (Figure 4). Therefore, in some of the embodiments of the pharmaceutical preparation of the present invention, a drug is loaded into a layer-by-layer film such that the drug is penetrated through the entire layer-by-layer film.

Additionally, the layer of the drug 14 may further be covered with a drug release control film so as to dramatically prolong the duration of drug action. Examples of the drug release control film include hydrophobic biodegradable films made of polyvinyl acetate, polylactic acid, polyglycolic acid, polycaprolactone, a copolymer thereof or the like.

The drug release control film may be separated from the layer-by-layer thin film of the present invention in advance so that the drug release control film is applied to the eye first and then the drug-loaded layer-by-layer thin film is applied thereon. By doing so, the layer of the drug 14 can further be covered with the drug release control film. Alternatively, the layer-by-layer thin film may be applied to the eye in advance and then the drug release control film may be applied thereon, or these procedures can be employed in combination.

Although one or more types of drugs that are to be loaded onto a layer-by-layer thin film of the present invention may be one or two or more types (e.g., two, three, four or five types, and preferably two types) of drugs, it is preferably one type of drug. When two or more types of drugs are to be loaded onto the layer-by-layer thin film of the present invention: all of the drugs may be layered on the surface layer; some of the drugs may be layered on the surface layer while the rest of the drugs are layered between the layers; or all of the drugs may be layered between the layers. Two or more types of drugs may be layered on the same surface layer or between the same layers, or they may be layered on separate surface layers or between separate layers.

Since in a preferable embodiment of a pharmaceutical preparation of the present invention, a drug to be contained is not fixed to a layer-by-layer thin film via a chemical bond, a broad range of drugs can be loaded onto the layer-by-layer thin film.

Examples of one or more types of drugs include one or more types of drugs that have conventionally been used as external medicines to be administered to eyes: for example, drugs conventionally used as drops (e.g., drugs for treating glaucoma, antimicrobial agents, steroid drugs, etc.), lachrymal secretion stimulating agents, anti-inflammatory agents, anti-allergic agents and the like, preferably drugs for treating glaucoma, antimicrobial agents, anti-inflammatory agents and the like, and more preferably drugs for treating glaucoma. Examples of one or more types of drugs for treating glaucoma include at least one or more types of those selected from a group consisting of prostaglandins, beta blockers and carbonic anhydrase inhibitors, and it is preferably prostaglandins. Examples of prostaglandins include Latanoprost, Bimatoprost, isopropyl unoprostone, travoprost, tafluprost, as well as Xalacom (R) and DuoTrav (R), i.e., combination agents of a prostaglandin and a beta blocker, and it is preferably Latanoprost.

In a pharmaceutical preparation of the present invention, one of these drugs may be used alone or two or more of them may be used in appropriate combination in the same pharmaceutical preparation.

A film thickness of a layer-by-layer thin film of the present invention before loading the drug is, for example, 500 nm or less, preferably 5 nm to 500 nm, more preferably 10 nm to 200 nm, and still more preferably 15 nm to 100 nm. A film thickness of a single drug layer is, for example, 10 nm to 400 nm, and more preferably 50 nm to 300 nm, where one or more, preferably one, of which is loaded onto a layer-by-layer thin film of the present invention. When a drug is loaded onto the layer-by-layer thin film of the present invention, the entire film thickness is, for example, 50 nm to 1000 nm, preferably 40 nm to 800 nm, more preferably 50 nm to 500 nm, and more preferably 60 nm to 400 nm.

A film thickness of the drug release control film is, for example, 5 nm to 500 nm and more preferably 50 nm to 200 nm.

A film thickness of the supporting film is, for example, 1 µm to 100 µm and more preferably 10 µm to 30 µm.

A film thickness of the protective film is, for example, 1 µm to 1000 µm, and more preferably 50 µm to 500 µm.

The size of a layer-by-layer thin film of the present invention may appropriately be selected according to the application. In the case where the layer-by-layer thin film of the present invention is applied to a human eye, for example, it is a quadrangle with a side length of 0.4 cm to 3.0 cm (preferably 0.5 cm to 2.0 cm), a circle with a diameter of 0.4 cm to 3.0 cm (preferably 0.5 to 2.0 cm), an oval with a length of the major axis of 0.4 cm to 3.0 cm (preferably 0.5 cm to 2.0 cm) and a length of the minor axis of 0.3 cm to 2.5 cm (preferably 0.4 cm to 1.5 cm), a doughnut shape with a diameter of 6 mm and a diameter of a central hollow of 3 mm, or the like.

The layer-by-layer thin film of the present invention is loaded with a drug in an amount of, for example, 0.25 µg/cm² to 25 µg/cm², and preferably 0.95 µg/cm² to 5 µg/cm².

A preferable embodiment of a pharmaceutical preparation of the present invention has a very thin layer-by-layer thin film and is superior in extensibility and deformability. Accordingly, concerns upon wearing such as mattering perceived by the wearer and adverse effects such as bloodshot are unlikely to be caused. Moreover, a more preferable embodiment of a pharmaceutical preparation of the present invention has high oxygen permeability and less invasive to the ocular tissue.

### 3. Method for preparing drug-loaded layer-by-layer thin film

A layer-by-layer thin film of the present invention can be formed by alternately layering polyelectrolyte (polycation and polyanion) layers with opposite charges on an appropriate substrate.

Examples of the substrate include a silicon substrate, a plastic substrate and a glass substrate, and preferably a plastic substrate. The substrate may have a curved surface that fits the curvature of an eyeball.

A layering method is not limited but preferably it is a method of alternately layering polyelectrolyte layers with opposite charges by a known film formation technique such as a spin coating process, a dipping process, a spray coating process or the like.

Figure 2 shows an exemplary method for preparing a drug-loaded layer-by-layer thin film by a spin coating process.

In the case where a spin coating process is used, for example, a predetermined concentration (e.g., 0.1 to 100 mg/mL, preferably 0.2 to 50 mg/mL, more preferably 0.3 to 20 mg/mL) of a polyelectrolyte (chitosan in Figure 2) is applied to a substrate with a spin coater at 100 to 10000 rpm for 1 to 60 seconds (4500 rpm for 15 seconds in Figure 2), and then the substrate is directly rotated for 10 to 60 seconds to dry, thereby forming a polyelectrolyte layer (Figure 2(a)). Next, a polyelectrolyte (sodium alginate in Figure 2) layer with an opposite charge is formed thereon by the same process (Figure 2(b)). In this manner, polyelectrolyte layers with opposite charges are alternately formed (Figure 2(c)).

In the case where a spin coating process is employed, the thickness of each of the polyelectrolyte layers can be controlled by changing the concentration of the polyelectrolyte solutions, the rotational speed of the spin coater, the rotational time of the spin coater, the temperature, the humidity or the like. Specifically, the thickness of the layer can be made thinner by lowering the concentrations of the polyelectrolyte solutions, by increasing the rotational speed of the spin coater, by extending the rotational time of the spin coater, by increasing the temperature, by increasing the humidity or the like.

In the case where a dipping process is employed, a substrate is immersed in a polyelectrolyte (e.g., chitosan) solution for a predetermined time to apply the solution to the substrate surface, and then the substrate immersed into a wash solution to remove the excess polyelectrolyte. Then, the substrate is immersed into a polyelectrolyte (e.g., sodium alginate) solution with an opposite charge for a predetermined time to apply the solution to the substrate surface and then the substrate is immersed into a wash solution to remove the excess polyelectrolyte. In this manner, polyelectrolyte layers with opposite charges are alternately formed. The thickness of each electrolyte layer may be controlled by changing the concentration or viscosity of each electrolyte solution, the immersion time, the immersion temperature or the like. The thickness of the layer can be made thinner by lowering the concentration or the viscosity, by shortening the immersion time, by increasing the immersion temperature or the like.

In the case where a spray coating process is employed, a polyelectrolyte (e.g., chitosan) solution is sprayed onto a substrate under predetermined spraying conditions for a predetermined time to apply the solution to the substrate surface, and then a wash solution is sprayed onto the substrate for a predetermined time to remove the excess polyelectrolyte. Subsequently, a polyelectrolyte (e.g., sodium alginate) solution with an opposite charge is sprayed onto the substrate under predetermined spraying conditions for a predetermined time to apply the solution to the substrate surface, and thereafter a wash solution is sprayed onto the substrate for a predetermined time to remove the excess polyelectrolyte. In this manner, polyelectrolyte layers with opposite charges are alternately formed. The thickness of each electrolyte layer can be controlled by changing the concentration or viscosity of each electrolyte solution, the spraying time, the washing time or the like. The thickness of the layer can be made thinner by lowering the concentration or the viscosity, by decreasing the size of the spray droplet, by shortening the spraying time, by extending the washing time or the like.

As shown in Figure 2(d), a method for preparing a layer-by-layer thin film of the present invention may further comprise a step of forming an additional layer (polyvinyl alcohol in Figure 2). The additional layer may be formed by dropping polyvinyl alcohol or the like and subsequently performing spin coating and drying. The method for forming an additional layer is not limited thereto, and an additional layer may be formed, for example, by drying after bar-coating, drying after immersion or the like.

According to the above-described method, the layer-by-layer thin film of the present invention can be prepared.

Next, a method for loading a drug onto the layer-by-layer thin film prepared as described above will be described.

First, the prepared layer-by-layer thin film is peeled off from the substrate (Figure 2(e)), and the layer-by-layer thin film is turned over (Figure 2(f)).

Then, a drug (Latanoprost in Figure 2) is dropped onto the surface of the layer-by-layer thin film and dried (Figure 2(g)), thereby forming a drug-loaded layer-by-layer thin film (Figure 2(h)). A method for loading a drug is not limited to dropping of a drug solution, and a drug may also be loaded onto a thin film, for example, by spin coating a drug solution, by spray coating a drug solution, by bar-coating a drug solution, or the like.

Although the method shown in Figure 2 is described by exemplifying a method for preparing a layer-by-layer thin film that has a drug loaded onto its surface, the present invention is not limited thereto. A layer-by-layer thin film having a drug between the layers can be prepared. A layer-by-layer thin film having a drug between the layers can also be prepared by inserting a step of loading a drug between the layering steps (Figures 2(b) to (c)). As described above, the step of loading a drug is performed, for example, by dropping and drying a drug, by subjecting a drug solution to spin coating, by subjecting a drug solution to spray coating, by subjecting a drug solution to bar-coating or the like, further repeating the layer-by-layer process, or forming a drug release control film by a spin coating process, a spray coating process or a bar-coating process.

A supporting film, a protective film and a drug release control film may be provided on the layer-by-layer thin film of the present invention, for example, by a spin coating process, a spray coating process or a bar-coating process. Alternatively, a film prepared in advance may be compressed to the layer-by-layer film of the present invention. The protective film may be provided by simply overlaying it on the layer-by-layer film or on a film obtained by combining the layer-by-layer thin film with various films.

As described above, the drug release control film may be separated from the layer-by-layer thin film of the present invention in advance, in which case the drug release control film is applied beforehand and then the drug-loaded layer-by-layer thin film is applied thereto. Alternatively, the layer-by-layer thin film may be applied to the eye beforehand and then the drug release control film may be applied thereto, or both procedures may be employed in combination.

### 4. Pharmaceutical preparation

A pharmaceutical preparation of the present invention comprises a layer-by-layer thin film and a drug loaded onto said thin film. The pharmaceutical preparation of the present invention is an external medicine which may be used, for example, for instilling a drug into the eye, the ocular surface, the periocular area such as the eyelid or the like. Preferably, it is used for instilling a drug into the eye.

According to the present invention, a drug is instilled, for example, by applying a thin-film pharmaceutical preparation to the eye.

In the case where the pharmaceutical preparation the present invention is to be used for instilling a drug into the eye, examples of the drugs include drugs that have conventionally been administered as eyedrops, such as drugs for treating glaucoma, antimicrobial agents, anti-inflammatory agents and anti-allergic agents. Preferably, the drug is a drug for treating glaucoma. A drug for treating glaucoma contains, for example, a prostaglandin, an autonomic agent such as a beta blocker, a carbonic anhydrase inhibitor, an ophthalmic therapeutic agent such as a cytoskeletal regulator as an active element, and preferably contains a prostaglandin as an active element. Examples of prostaglandins include Latanoprost, Bimatoprost, isopropyl unoprostone, travoprost, tafluprost and Xalacom (R) and DuoTrav (R), i.e., combination agents of a prostaglandin and a beta blocker, and preferably Latanoprost.

Other than the active element, these drugs may also contain a pharmaceutically acceptable carrier or additive. Examples of such carriers and additives include water, pharmaceutically acceptable organic solvents, collagen, polyvinyl alcohol, polyvinylpyrrolidone, carboxy vinyl polymer, carboxymethylcellulose sodium, sodium polyacrylate, sodium alginate, water-soluble dextran, carboxymethyl starch sodium, pectin, methylcellulose, ethylcellulose, xanthane gum, gum arabic, casein, agar, polyethylene glycol, diglycerin, glycerin, propylene glycol, vaseline, paraffin, stearyl alcohol, stearic acid, human serum albumin, mannitol, sorbitol, lactose, and surfactants acceptable as pharmaceutical additives.

A dosage of a pharmaceutical preparation of the present invention differs depending on the age, sex, weight, type of disorder, conditions, number of doses and the like. For example, when it is used for treating an adult (weight: 60kg) with glaucoma, a pharmaceutical preparation that is loaded with 0.45 µg to 5 µg (0.45 µg/cm² to 5 µg/cm²), preferably 0.95 µg to 5 µg (0.95 µg/cm² to 5 µg/cm²) of prostaglandin per cm² thin film is applied to the eye, for example, once in two to thirty days, preferably once in three to twenty days, and more preferably once in seven to twenty days.

A preferable embodiment of a pharmaceutical preparation of the present invention has lower cellular toxicity and longer duration of action with a single dose.

Since a preferable embodiment of a pharmaceutical preparation of the present invention has a longer duration of action with a single dose, for example, a doctor can give a dose of the pharmaceutical preparation of the present invention to a patient at every hospital visit so that the patient has no need of self-administration.

In addition, the present invention also comprises a method for treating a disease by administering a pharmaceutical preparation of the present invention to a subject in need of the treatment of a disease. The subject is a human, a non-human mammal (mouse, rat, rabbit, dog, cat, etc) or the like, and preferably a human. The disease is, for example, an ocular disease (e.g., glaucoma, keratitis, uveitis, etc.), conjunctivitis or the like, and preferably glaucoma. A dosage and the like of the pharmaceutical preparation is as described above.

In order to administer two or more types of drugs to a patient, a single type of pharmaceutical preparation containing two or more types of drugs may be administered to a subject or two or more types of pharmaceutical preparations each containing a single type of drug may be administered to the subject.

In some embodiments of a pharmaceutical preparation of the present invention, as described above, a supporting film layer is included on a surface of a layer-by-layer thin film that is opposite to the drug-layered surface. In this case, the pharmaceutical preparation may be administered by bringing the drug-layered surface into contact with an eye, and thereafter removing the supporting film on the opposite surface. When the supporting film is water-soluble like polyvinyl alcohol, the supporting film can be removed by washing with physiological saline or the like. Alternatively, it may be left as it is so as to be naturally removed with tears.

Furthermore, the present invention provides use of a drug-loaded layer-by-layer thin film for producing a pharmaceutical preparation. The pharmaceutical preparation and the drug-loaded layer-by-layer thin film are as described above.

### EXAMPLES

Hereinafter, the present invention will be described more specifically by way of examples, although the present invention should not be limited to these examples.

### Example 1: Method for preparing drug-loaded layer-by-layer (LbL) nanosheet

All of the operations were performed with a spin coater (Opticoat MS-A 150, MIKASA) placed in a clean room (Class 10,000). A silicon substrate (produced by KST World) was cut into 2 cm x 2 cm, immersed in a sulfuric acid/hydrogen peroxide water (3/1, v/v) for 10 minutes and washed with deionized water (resistivity 18MQcm).

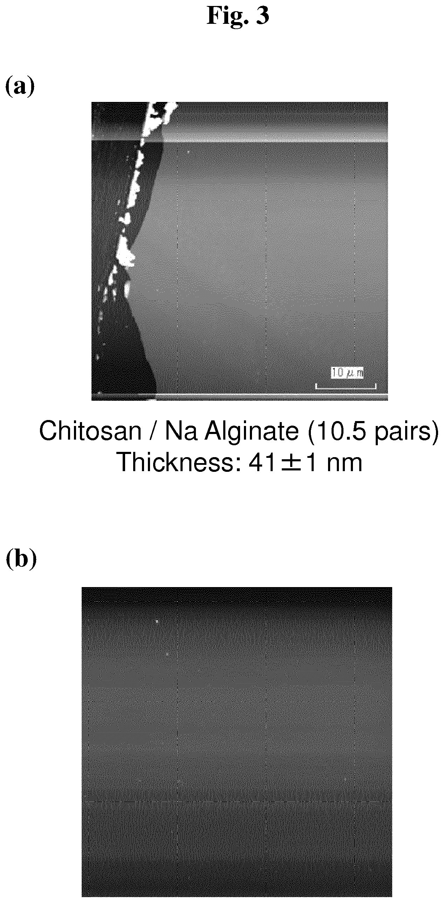

This substrate was placed in a spin coater, onto which a chitosan solution (Mw:88kD, Nacalai Tesque, 1mg/mL, 1 v/v % acetic acid/0.5M aqueous NaCl solution) was dropped for 150 µL. The resultant was subjected to spin coating (4500 rpm, 15 seconds), after which the substrate was washed twice with deionized water and directly rotated for 30 seconds to dry (Figure 2(a)). Subsequently, a sodium alginate solution (Mw:106 kD, Nacalai Tesque, 1 mg/mL, 0.5M aqueous NaCl solution) was dropped for 150 µL. The resultant was subjected to spin coating (4500 rpm, 15 seconds), after which the substrate was washed twice with deionized water and directly rotated for 30 seconds to dry (Figure 2(b)). Spin coatings of chitosan and sodium alginate were repeated to prepare a LBL nanosheet having 10.5 pairs (21 layers) of them (Figure 2(c)). The film thickness was measured with an atom force microscope (AFM) (NanoScale Hybrid Microscope, Keyence, tapping mode) and found to be 41±1 nm (Figure 3).

0.5 mL of an aqueous polyvinyl alcohol (PVA) solution (Mw:22,000, Kanto Chemical, 100 mg/mL) was dropped onto the LbL nanosheet and dried, thereby forming a PVA film on the LbL nanosheet (room temperature, overnight) (Figure 2(d)). The LbL nanosheet, together with the PVA film, was peeled off from the silicon substrate (Figure 2(e)), and applied to another silicon substrate with the PVA film facing down (Figure 2(f)). 10 µL of a Latanoprost solution (Mw:432.6, SIGMA, 1 mg/mL, methanol) was dropped on the LbL nanosheet as the upper surface and dried (Figure 2(g)), thereby producing a nanosheet loaded with Latanoprost in an amount of 2.5 µg/cm² (Figure 2(h)). When observed with AFM, Latanoprost was loaded such that Latanoprost aggregation with an average thickness of 248 ± 58 nm and an average size of 6.2 ± 1 µm was confirmed to be loaded and thus a Latanoprost-loaded LBL nanosheet was prepared (Figure 4). The Latanoprost-loaded LbL nanosheet, together with PVA, was peeled off from the silicon substrate and subjected to the following example.

### Example 2: Measurement of release behavior of Latanoprost-loaded LbL nanosheet (1)

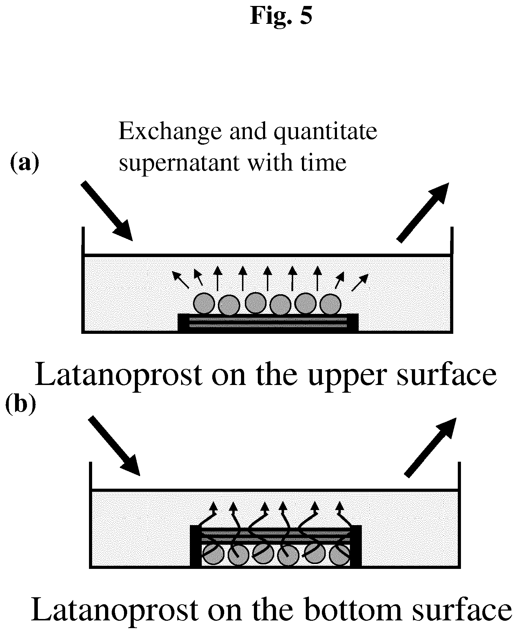

The Latanoprost-loaded LbL nanosheet (1x1 cm²) prepared according to the method of Example 1 was applied to a well plate (6-well plate [flat bottom], P06F01S, Stem) with the Latanoprost-loaded surface either facing down or up (Figures 5(a) and 5(b)). The sides were closed with a tape so as to prevent leak from the sides. To this, 5 mL of physiological saline was added for immersing the Latanoprost-loaded LbL nanosheet therein. After 30 minutes of immersion, the physiological saline in the plate was entirely collected, and another 5 mL of physiological saline was added for reimmersion. This collection was repeated 1, 2, 3, 6 and 24 hours following the initial immersion. The collected specimens were quantified with a microplate reader (measurement wavelength: λ = 405-420 nm) using Latanoprost EIA kit (Cayman Chemical Item Number 516811). When Latanoprost was on the upper surface, the entire surface of Latanoprost was released in about 30 minutes (Figure 6(a)) whereas when Latanoprost was on the bottom surface, Latanoprost was slowly released and almost the whole amount was released in about 24 hours (Figure 6(b)).

### Example 3: Evaluation of pharmacological behavior of Latanoprost-loaded LbL nanosheet

The pharmacological effect was evaluated by using rats (Charles River, Wistar).

The Latanoprost-loaded or Latanoprost-unloaded LBL nanosheets (about 3x3 mm) produced according to the method of Example 1 were applied to rat corneas. Intraocular pressures were measured before the application and 1, 2, 3, 4, 5, 6, 7, 8, 9, 10 and 17 days after the application.

Here, the intraocular pressures were measured with TonoLab, a pressure measuring device for small animals from ICARE FINLAND. Measurements that were judged to be highly reliable by automatic reliability judgment were repeated in triplicate to obtain an average thereof.

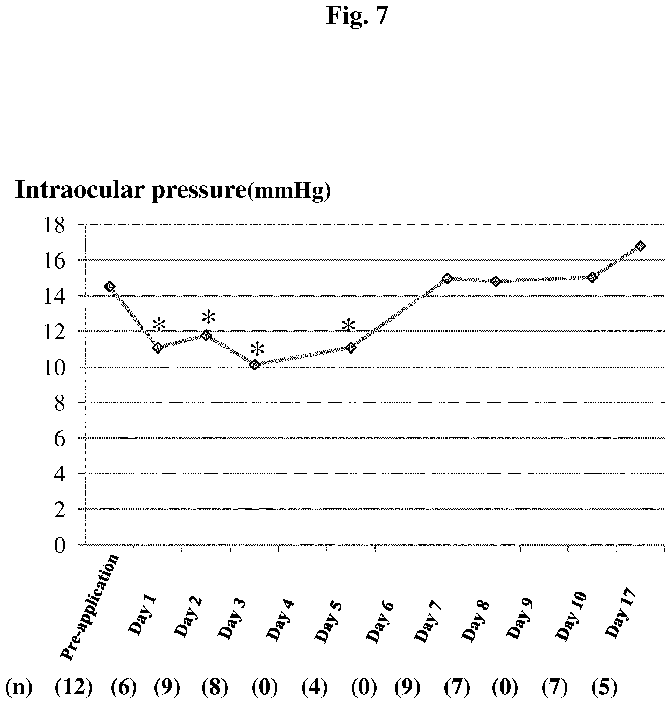

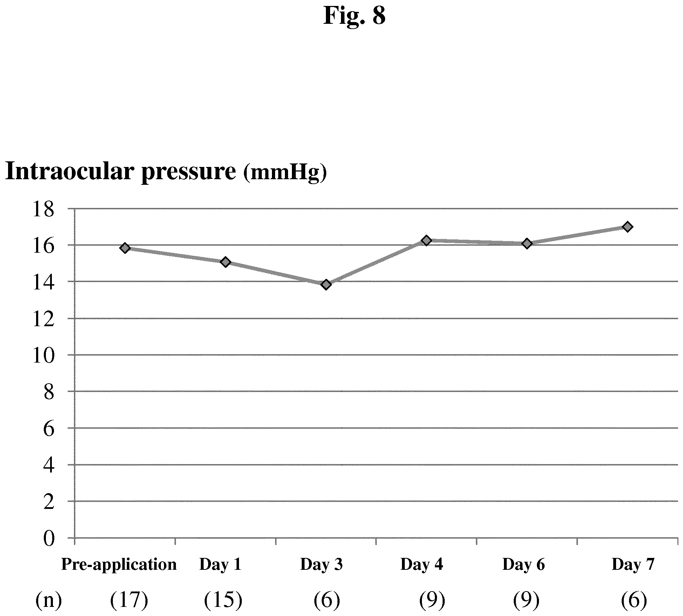

As a result, when the Latanoprost-loaded LBL nanosheets was used, the intraocular pressures of rats were significantly decreased on Days 1 to 5 as compared to those with the Latanoprost-unloaded LBL nanosheets (Figures 7 and 8). Specifically, the Latanoprost-loaded LBL nanosheet was able to sustain the action of Latanoprost to lower the intraocular pressure for 5 days. On the other hand, when Latanoprost was applied to the eyes in a form of eyedrops, the action of Latanoprost to lower the intraocular pressure only sustained for about a day as described in the written material from Pfizer. Accordingly, use of the Latanoprost-loaded LBL nanosheet can greatly prolong the action of Latanoprost to lower the intraocular pressure as compared to the use of eyedrops.

### Example 4: Evaluation of tissue toxicity of Latanoprost-loaded LbL nanosheet

Tissue toxicity was evaluated using a Wistar rat from Charles River and a New Zealand White rabbit from Oriental Yeast Co., Ltd.

The Latanoprost-loaded or Latanoprost-unloaded nanosheet produced according to the method of Example 1 were applied to the corneas and conjunctivas of the rat or the rabbit. One, three and seven days after the nanosheet application, the tissue toxicities were examined with a slit-lamp microscope, i.e., one type of stereoscopic microscope that is frequently used for ophthalmologic examination.

At any time points after the nanosheet application, both rat and rabbit had the following results.

Bloodshot of the ocular surface, corneal disorder, anterior chamber inflammation or the like was not found in the eyes applied with the nanosheets (Figures 9(a) and 9(b)). Here, Figure 9(a) shows the rabbit corneal specimen five days after the application of the Latanoprost-loaded LBL nanosheet while Figure 9(b) shows the rat corneal specimen seven days after the application of the Latanoprost-loaded LBL nanosheet.

The behaviors of the rat and the rabbit were observed after the nanosheet application. There was no change in the behavior that seems to be caused by the nanosheet application. Since the rat and the rabbit did not show any scratching motion, the nanosheets were considered to provide sufficiently comfortable wearing for these animals. This was considered to be due to the very thin nanosheet that allows the sheet to be buried in the tear layers so that the animals can feel no mattering, and due to the high oxygen permeability of the nanosheet that allows less invasion into the ocular tissue.

These results were the same for both Latanoprost-loaded and Latanoprost-unloaded nanosheets.

### Example 5: Evaluation of pharmacological behavior of Latanoprost-loaded LbL nanosheet

A Latanoprost-loaded LBL nanosheet (0.25 cm²) produced according to the method of Example 1 was applied to a white rabbit. Then, one, three and seven days after the application, the aqueous humors were collected. The aqueous humors were suctioned by inserting a 30-gauge needle provided on a 1 mL syringe into the anterior chamber from the corneoscleral limbus while carefully avoiding a liquid substance such as tears from the ocular surface to enter. The amount to be collected was about 0.15 mL in average.

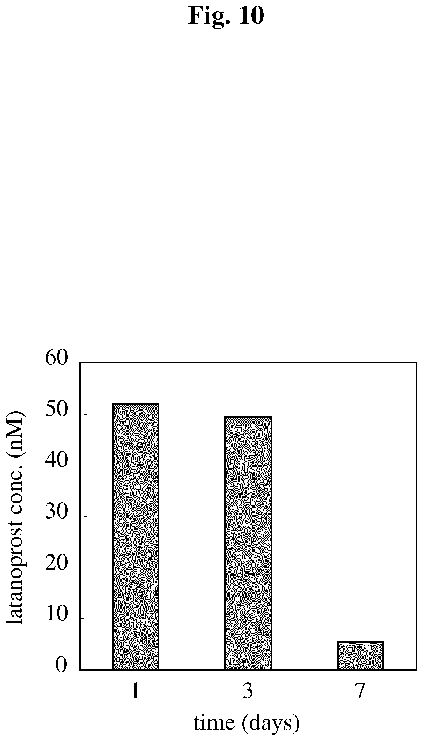

The concentrations of the collected specimens were measured with a microplate reader (measurement wavelength: λ = 405-420 nm) using Latanoprost EIA kit (Cayman Chemical Item Number 516811) (Figure 10). One day (24 hours) and three days (72 hours) after the application, the Latanoprost concentrations were 52.1 nM and 49.4 nM, respectively, while the Latanoprost concentration seven days after the application was 5.5 nM. In the case of Latanoprost eyedrops currently in clinical use, the drug concentration in the aqueous humor of the eye 24 hours after instillation of the eyedrops was reported to be about 0.5 nM, which was lower than that of the Latanoprost-loaded LBL nanosheet by about 1/100, and after 24 hours became lower than that of the lower detection limit. Accordingly, since the Latanoprost-loaded LBL nanosheet allows sustained release of a drug from the nanosheet to the eye for a long period of time as compared to conventional eyedrops, it allows a high drug concentration to be maintained in the anterior chamber for a very long time and therefore the action of lowering the intraocular pressure is considered to sustain for a long period of time.

As described in the above examples, a Latanoprost-containing nanosheet was capable of prolonging the duration of Latanoprost action to lower the intraocular pressure with a single application without exhibiting apparent cellular toxicity on the ocular surface. Hence, a preferable embodiment of a pharmaceutical preparation of the present invention was proved to have a longer duration of drug action with a single dose.

## Claims

1. A pharmaceutical preparation for use in a method of treating glaucoma by application to the eye, said preparation comprising a layer-by-layer thin film that is producible by alternately layering a polycation and a polyanion, and one or more type(s) of drug loaded onto the layer-by-layer thin film or between the layers, which is/are to be instilled into the eye, the ocular surface, or the periocular area; wherein
the one or more type(s) of drug is/are for treating glaucoma; and
wherein the polycation is selected from the group consisting of chitosan, polylysine, polyarginine, polyhistidine, ionene, poly(quarterized pyridine), polymers of diallyldiakylammonium salt; and
wherein the polyanion is selected from the group consisting of alginic acid, hyaluronic acid, chondroitin sulfate, polyglutamic acid, polymethacrylic acid, polyacrylic acid, polystyrene sulfonate and alkali salts thereof.

2. The pharmaceutical preparation for use according to Claim 1, wherein the polycation is chitosan.

3. The pharmaceutical preparation for use according to claim 1 or claim 2, wherein the polyanion is sodium alginate.

4. The pharmaceutical preparation for use according to any one of Claims 1 to 3, wherein the layer-by-layer thin film has a film thickness of 5 nm to 500 nm without the drug(s).

5. The pharmaceutical preparation for use according to any one of Claims 1 to 4, wherein the number of polycation and polyanion layers of the layer-by-layer thin film is 2 to 50.

6. The pharmaceutical preparation for use according to any one of Claims 1 to 5, wherein the shape of the layer-by-layer thin film is a quadrangle, a circle, an oval, a doughnut shape or a ring shape.

7. The pharmaceutical preparation for use according to any one of Claims 1 to 6, wherein at least one drug is loaded by being layered onto one surface of the layer-by-layer thin film.

8. The pharmaceutical preparation for use according to any one of Claims 1 to 7, wherein a drug is loaded at an amount of 0.25 µg/cm² to 25 µg/cm².

9. The pharmaceutical preparation for use according to Claim 1, wherein the one or more type(s) of drug for treating glaucoma is/are at least one type of drug selected from a group consisting of prostaglandins, beta blockers and carbonic anhydrase inhibitors.

10. The pharmaceutical preparation for use according to Claim 9, wherein the one or more type(s) of drug for treating glaucoma include or consist of Latanoprost.

11. The pharmaceutical preparation for use according to any one of Claims 1 to 10, wherein the layer-by-layer thin film further comprises a polyvinyl alcohol layer as a supporting film layer on at least one surface thereof.

12. The pharmaceutical preparation for use according to Claim 11, comprising the supporting film layer on the surface opposite to the surface layered with the drug.

13. The pharmaceutical preparation for use according to Claim 12, which is for use by applying it to the eyeball by bringing the drug-layered surface into contact with the eye and then removing the supporting film on the opposite surface.

14. The pharmaceutical preparation for use according to any one of Claims 7 to 13, further comprising, on the drug-layered surface, a drug release control film formed from polyvinyl acetate, polylactic acid, polyglycolic acid, polycaprolactone or a copolymer of any combination thereof.

15. The pharmaceutical preparation for use according to any one of Claims 1 to 13, which is for use in a method in which a drug control film formed from polyvinyl acetate, polylactic acid, polyglycolic acid, polycaprolactone or a copolymer of any combination thereof is applied to the eye, and then the pharmaceutical preparation is applied on said drug control film.

## Patentansprüche

1. Pharmazeutisches Präparat zur Verwendung in einem Verfahren zur Behandlung von Glaukomen durch Anwendung auf das Auge, wobei das Präparat einen geschichteten Dünnfilm, der durch abwechselndes Schichten eines Polykations und eines Polyanions herstellbar ist, und eine oder mehrere Arten eines Arzneimittels umfasst, das auf den geschichteten Dünnfilm oder zwischen die Schichten geladen ist, das/die in das Auge, auf die Augenoberfläche oder in den periokularen Bereich einzutropfen ist/sind; wobei
die eine oder mehrere der Arten des Arzneimittels zur Behandlung von Glaukomen dient/dienen; und
wobei das Polykation aus der aus Chitosan, Polylysin, Polyarginin, Polyhistidin, Ionen, Poly(quarterniertes Pyridin), Polymeren von Diallyldialkylammoniumsalz bestehenden Gruppe ausgewählt ist; und
wobei das Polyanion aus der aus Alginsäure, Hyaluronsäure, Chondroitinsulfat, Polyglutaminsäure, Polymethacrylsäure, Polyacrylsäure, Polystyrolsulfonat und Alkalisalzen davon bestehenden Gruppe ausgewählt ist.

2. Pharmazeutisches Präparat zur Verwendung nach Anspruch 1, wobei das Polykation Chitosan ist.

3. Pharmazeutisches Präparat zur Verwendung nach Anspruch 1 oder Anspruch 2, wobei das Polyanion Natriumalginat ist.

4. Pharmazeutisches Präparat zur Verwendung nach einem der Ansprüche 1 bis 3, wobei der geschichtete Dünnfilm eine Filmdicke von 5 nm bis 500 nm ohne das Arzneimittel/die Arzneimittel aufweist.

5. Pharmazeutisches Präparat zur Verwendung nach einem der Ansprüche 1 bis 4, wobei die Anzahl von Polykation- und Polyanion-Schichten des geschichteten Films 2 bis 50 beträgt.

6. Pharmazeutisches Präparat zur Verwendung nach einem der Ansprüche 1 bis 5, wobei die Form des geschichteten Dünnfilms ein Viereck, ein Kreis, ein Oval, eine Doughnut-Form oder eine Ringform ist.

7. Pharmazeutisches Präparat zur Verwendung nach einem der Ansprüche 1 bis 6, wobei zumindest ein Arzneimittel durch Schichten auf eine Oberfläche des geschichteten Dünnfilms aufgeladen wird.

8. Pharmazeutisches Präparat zur Verwendung nach einem der Ansprüche 1 bis 7, wobei ein Arzneimittel in einer Menge von 0,25 µm/cm² bis 25 µg/cm² aufgeladen wird.

9. Pharmazeutisches Präparat zur Verwendung nach Anspruch 1, wobei die eine Art oder die mehreren Arten eines Arzneimittels zur Behandlung von Glaukomen zumindest eine Art von Arzneimittel ist/sind, das/die aus einer aus Prostaglandinen, Betablockern und Carbonsäureanhydrase-Inhibitoren bestehenden Gruppe ausgewählt ist/sind.

10. Pharmazeutisches Präparat zur Verwendung nach Anspruch 9, wobei die eine Art oder die mehreren Arten eines Arzneimittels zur Behandlung von Glaukomen Latanoprost umfassen oder daraus bestehen.

11. Pharmazeutisches Präparat zur Verwendung nach einem der Ansprüche 1 bis 10, wobei der geschichtete Dünnfilm weiters eine Polyvinylalkoholschicht als Trägerfilmschicht auf zumindest einer Oberfläche davon umfasst.

12. Pharmazeutisches Präparat zur Verwendung nach Anspruch 11, umfassend die Trägerfilmschicht auf der der mit dem Arzneimittel beschichteten Oberfläche entgegengesetzten Oberfläche.

13. Pharmazeutisches Präparat zur Verwendung nach Anspruch 12, das zur Verwendung durch Anwendung davon auf den Augapfel durch In-Kontakt-Bringen der mit dem Arzneimittel beschichteten Oberfläche mit dem Auge und dann Entfernen des Trägerfilms auf der entgegengesetzten Oberfläche dient.

14. Pharmazeutisches Präparat zur Verwendung nach einem der Ansprüche 7 bis 13, weiters umfassend einen Arzneimittelfreisetzungskontrollfilm, der aus Polyvinylacetat, Polymilchsäure, Polyglykolsäure, Polycaprolacton oder einem Copolymer einer beliebigen Kombination daraus gebildet ist, auf der mit dem Arzneimittel beschichteten Oberfläche.

15. Pharmazeutisches Präparat zur Verwendung nach einem der Ansprüche 1 bis 13, das zur Verwendung in einem Verfahren dient, in dem ein Arzneimittelkontrollfilm, der aus Polyvinylacetat, Polymilchsäure, Polyglykolsäure, Polycaprolacton oder einem Copolymer einer beliebigen Kombination daraus gebildet ist, auf das Auge angewendet wird und wobei das pharmazeutische Präparat dann auf den Arzneimittelkontrollfilm aufgebracht wird.

## Revendications

1. Préparation pharmaceutique destinée à être utilisée dans un procédé de traitement du glaucome par application sur l'œil, ladite préparation comprenant un film mince déposé couche par couche qui peut être produit par stratification en alternance d'un polycation et d'un polyanion, et un ou plusieurs types de médicament chargés sur le film mince déposé couche par couche, qui doivent être instillés dans l'œil, la surface oculaire ou la zone périoculaire ; dans laquelle
les types de médicament sont destinés à traiter un glaucome ; et
dans laquelle le polycation est choisi dans le groupe constitué par le chitosane, la polylysine, la polyarginine, la polyhistidine, l'ionène, la poly(pyridine quarternisée), les polymères de sel de diallyldiakylammonium ; et
dans laquelle le polyanion est choisi dans le groupe constitué par l'acide alginique, l'acide hyaluronique, le sulfate de chondroïtine, l'acide polyglutamique, l'acide polyméthacrylique, l'acide polyacrylique, le polystyrène sulfonate et des sels alcalins de ceux-ci.

2. Préparation pharmaceutique à utiliser selon la revendication 1, dans laquelle le polycation est le chitosane.

3. Préparation pharmaceutique à utiliser selon la revendication 1 ou la revendication 2, dans laquelle le polyanion est l'alginate de sodium.

4. Préparation pharmaceutique à utiliser selon l'une quelconque des revendications 1 à 3, dans laquelle le film mince déposé couche par couche a une épaisseur de film de 5 nm à 500 nm sans le ou les médicaments.

5. Préparation pharmaceutique à utiliser selon l'une quelconque des revendications 1 à 4, dans laquelle le nombre de couches de polycation et de polyanion du film mince déposé couche par couche est de 2 à 50.

6. Préparation pharmaceutique à utiliser selon l'une quelconque des revendications 1 à 5, dans laquelle la forme du film mince déposé couche par couche est un quadrilatère, un cercle, un ovale, une forme de beignet ou une forme d'anneau.

7. Préparation pharmaceutique à utiliser selon l'une quelconque des revendications 1 à 6, dans laquelle au moins un médicament est chargé en étant stratifié sur une surface du film mince déposé couche par couche.

8. Préparation pharmaceutique destinée à être utilisée selon l'une quelconque des revendications 1 à 7, dans laquelle un médicament est chargé à une quantité de 0,25 µg/cm² à 25 µg/cm².

9. Préparation pharmaceutique à utiliser selon la revendication 1, dans laquelle les un ou plusieurs types de médicament pour traiter un glaucome est/sont au moins un type de médicament choisi dans un groupe constitué de prostaglandines, de bêta-bloquants et d'inhibiteurs d'anhydrase carbonique.

10. Préparation pharmaceutique à utiliser selon la revendication 9, dans laquelle les un ou plusieurs types de médicament pour traiter un glaucome comprennent ou consistent en du latanoprost.

11. Préparation pharmaceutique à utiliser selon l'une quelconque des revendications 1 à 10, dans laquelle le film mince déposé couche par couche comprend en outre une couche d'alcool polyvinylique en tant que couche de film de support sur au moins une surface de celui-ci.

12. Préparation pharmaceutique à utiliser selon la revendication 11, comprenant la couche de film de support sur la surface opposée à la surface sur laquelle le médicament est stratifié.

13. Préparation pharmaceutique à utiliser selon la revendication 12, qui doit être utilisée en l'appliquant sur le globe oculaire en mettant la surface sur laquelle le médicament est stratifié en contact avec l'œil, puis en retirant le film de support sur la surface opposée.

14. Préparation pharmaceutique à utiliser selon l'une quelconque des revendications 7 à 13, comprenant en outre, sur la surface sur laquelle le médicament est stratifié, un film de commande de libération de médicament formé à partir d'acétate de polyvinyle, d'acide polylactique, d'acide polyglycolique, de polycaprolactone ou d'un copolymère de toute combinaison de ceux-ci.

15. Préparation pharmaceutique à utiliser selon l'une quelconque des revendications 1 à 13, qui est destinée à être utilisée dans un procédé dans lequel un film de commande de médicament formé d'acétate de polyvinyle, d'acide polylactique, d'acide polyglycolique, de polycaprolactone ou d'un copolymère de toute combinaison de ceux-ci est appliqué sur l'œil, puis la préparation pharmaceutique est appliquée sur ledit film de commande de médicament.
